# EUROPEAN PATENT APPLICATION

(11) **EP 2 682 069 A1**
(43) Date of publication of application: **08.01.2014**
(21) Application number: 12175041.8
(22) Date of filing: 05.07.2012
(51) Int. Cl.: A61B 19/02, B65D 37/00

(54) **A medical packaging system**

(71) Applicant: Unomedical A/S, 3460 Birkerød (DK)
(72) Inventor: Aakerlund, Karsten, 2970 Hørsholm (DK)
(74) Representative: Hoffmann Dragsted A/S

(57) **Abstract**

The present invention relates to a medical packaging system, comprising a medical packaging at least partly made of plastic and having a first inner face and a second inner face and an openable end, enclosing a medical article at least partly made of plastic and having a first surface and a second surface, further comprising a slip substantially made of a non-plastic material, arranged at least between the first surface of the medical article and the first inner face of the medical packaging. Further, the present invention relates to a method of producing a medical packaging system according to the present invention and to a method for extracting such medical article from the medical packaging system according to the invention.

## Description

### Field of the invention

The present invention relates to a medical packaging system. Further, the present invention relates to a method of producing a medical packaging system according to the present invention and to a method for extracting such medical article from the medical packaging system according to the invention.

### Background art

The number of elderly people is increasing around the world and more and more elderly people are treated at home. Some of the benefits associated with more elderly people staying in their own home longer is that they are in a familiar environment and that the costs associated with hospital care or nursing homes are reduced. At the same time, the elderly need to play a more active role in their treatment and this puts special requirements on the medical articles used by the elderly when treating themselves.

A common condition for which elderly people treat themselves is urinary problems. A urinary bag could then be a part of the treatment together with tubes and other accessories needed to connect the urinary bag to the urinary tract. Such articles need to be kept sterile or close to sterile and are therefore packaged in packaging that maintains the article sterile until used. The packaging must be easy to use even if a person has an age-associated problem such as, reduced muscle power, reduced gripping ability, shivering etc.

Currently, both the packaging for medical articles and e.g. a urine bag are made of plastic, and the urine bag and the packaging are furthermore made of flexible materials, making it very difficult for elderly or disabled people to extract the urine bag from the bag packaging.

### Summary of the invention

It is an object of the present invention to wholly or partly overcome the above disadvantages and drawbacks of the prior art. More specifically, it is an object to provide an improved packaging for medical articles in which the medical article is easier to extract for elderly and/or disabled people.

The above objects, together with numerous other objects, advantages, and features, which will become evident from the below description, are accomplished by a solution in accordance with the present invention by a medical packaging system, comprising:
- a medical packaging at least partly made of plastic and having a first inner face and a second inner face and an openable end, enclosing
- a medical article at least partly made of plastic and having a first surface and a second surface,
further comprising a slip substantially made of a non-plastic material, arranged at least between the first surface of the medical article and the first inner face of the medical packaging.

By arranging a slip substantially made of a non-plastic material between the medical article and the packaging of plastic material, the friction between the plastic materials of the medical article and the packaging material may be substantially reduced, making extraction of the medical article from the packaging much easier for the user.

In one embodiment, the slip may be connected with the medical article.

Also, the slip may be engaged with the medical article.

Moreover, the slip may be abutting the medical article.

By having the medical article connecting/engaging/abutting the slip, the user may choose to either grab and pull the slip or grab and pull the medical article to extract the medical article. When grabbing and pulling the article, the medical article slides on the slip reducing the friction between the first surface of the medical article and the first inner face of the medical packaging, thus reducing the friction to be overcome by the user when extracting the medical article. Alternatively, the user can grab and pull the slip. The slip then acts as a carrier to the medical article and extracts the medical article, together with the slip, out of the packaging.

In an embodiment, the slip may be a leaflet.

By using the accompanying leaflet as the slip, it is possible to reduce the extra costs of producing a slip.

Furthermore, the openable end may be hermetically closed by welding or adhering, such as glueing.

The slip described above may project from a first end of the medical article towards the openable end of the medical packaging.

By having the slip projecting from the edge of the medical article, an area of the slip is created which the user can grab and pull, hence aiding the user in getting a grip on the slip when extracting the slip and the medical article.

Also, the slip may be connected with the medical article by friction, and the friction between the slip and the medical article may be higher than the friction between the first surface and/or the second surface of the medical article and the first inner face and/or the second inner face of the medical packaging.

By connecting the medical article and the slip by means of friction, a simple connection between the slip and the medical article is achieved. By having a higher friction between the medical article and the slip than between the medical article and the medical packaging, the medical article follows the slip out of the medical packaging when the user grabs and pulls the slip.

In one embodiment, a friction between the slip and the medical article is lower than a friction between the first surface and/or second surface of the medical article and the first inner face and/or second inner face of the medical packaging.

By having lower friction between the slip and the medical article than between the medical article and the medical packaging, the medical article may be extracted by grabbing and pulling the medical article itself. This results in the user having to use less force when extracting the medical article than if he/she has to overcome the friction between the medical article and the medical packaging without the slip.

Said slip may be connected with the medical article by an adhesive or by means of welding.

By using a connection created by an adhesive or by a weld, a more secure connection between the medical article and the slip is created than if the medical article and the slip are connected by means of friction. This allows for a greater force to be used when pulling the slip and the slip to be pulled in more arbitrary directions compared to connecting the medical article and the slip by use of friction.

In another embodiment, the slip may be connected with the medical article by a mechanical attachment.

By using a mechanical attachment as the connection between the slip and the medical article, several types of locking connections may be incorporated with the slip and/or the medical article to further secure the slip to the medical article when the user grabs and pulls the slip to extract the medical article.

In one embodiment, the mechanical attachment may be a projection on a first surface of the slip facing the medical article, which projection is adapted to engage with a depression, a slit, or an opening on the medical article, or vice versa.

By making a depression, a slit or an opening in the slip and having a projection on the medical article, the medical article may be secured to the slip when the medical article and the slip are pulled out of the medical packaging. Additionally, the medical article and the slip may easily be separated after extraction by disengaging the projection and the slit.

Further, the slip may have a first end, a second end and a first surface facing the medical article and the mechanical attachment may be formed as a part of the second end of the slip and angled in relation to the first surface of the slip facing the medical article, with an angle of 0-90 degrees.

The mechanical attachment forming part of the second end of the slip, said mechanical attachment being angled in relation to the first surface of the slip, results in the slip having an elevated "edge" abutting the medical article when the user grabs and pulls the slip to extract the medical article.

Moreover, the mechanical attachment may comprise a first connection part and a second connection part, the first connection part may be arranged on the first surface of the slip facing the medical article, and the second connection part may be arranged on the first surface of the medical article, or vice versa, the first connection part having a shape corresponding to the shape of the second connection part.

The first connection part may be formed at the second end of the slip.

By using two connection parts which complement each other's shape, the shape of the mechanical attachment between the medical article and the slip may be arbitrarily chosen among a variety of different locking shapes.

Additionally, the slip may be releasably connected with the medical article.

Also, the connection between the slip and the medical article may be breakable.

By having a releasable or breakable connection, the slip may be separated from the medical article after extraction, so that the slip can be read more easily, thrown away or reused.

In an embodiment, the first surface of the slip facing the medical article may have an area, and the first surface of the medical article may have an area, the area of the slip covering at least 10% of the area of the first surface of the medical article, preferably at least 30%, more preferably at least 50%, and most preferably between 80% and 100%.

The slip may be folded or otherwise constructed to only cover part of the medical article so that the medical article is still visible through the packaging or in order to save production costs related to the slip.

Moreover, the slip may be partly arranged between the first inner face of the medical packaging and the first surface of the medical article and partly arranged between the second inner face of the medical packaging and the second surface of the medical article.

By arranging the slip underneath and on top of the medical article, the friction between the medical article and the medical packaging may be further reduced compared to the slip being arranged on one side only. Further, the slip partly envelops the medical article, allowing the slip to act as an extraction device so that when the slip is pulled, the medical article is extracted, together with the slip, out of the medical packaging without using friction, an adhesive agent or similar mechanical attachment between the slip and the medical article.

In an embodiment, the second surface of the medical article may have an area and the area of the first surface of the slip facing the medical article may cover at least 10% of the area of the second surface of the medical article, preferably at least 30%, more preferably at least 50%, and most preferably between 80% and 100%.

By allowing the slip to cover the medical article in an arbitrary way and to be arranged between the second inner face of the medical packaging and the second surface of the medical article, the slip may have different shapes partly covering the bottom and the top of the medical article.

Further, the medical article may have a first end and a second end, the first end of the medical article being arranged closer to the openable end of the medical packaging than the second end of the medical article and the slip being arranged partly covering the second end of the medical article.

By having the slip partly covering the second end of the medical article, the slip may be formed as a "hook" and the slip thereby acts an extraction device which is capable of securing the slip to the medical article at the second end of the medical article when the article is extracted.

The medical article may have a first side and a second side and the slip may be partly covering the first side and the second side of the medical article and partly covering the second end of the medical article.

By having the slip partly covering the two sides and partly covering the second end of the medical article, the slip may be formed as a pouch wherein the medical article may be retained which further secures the medical article to the slip when the slip is pulled in order to extract the medical article from the medical packaging.

In one embodiment, the slip may envelop the medical article.

By having the slip enveloping the medical article, the friction between the medical article and the medical packaging may be further reduced since the friction thus depends on the interaction between the medical packaging and the slip and not on the interaction between the medical article and the medical packaging. This allows for the slip to be made of a material providing low friction between the packaging and the slip so that the user can easily extract the medical article.

Also, a first part of the second end of the slip may be connected in a connection point with an intermediate part of the medical packaging, adjacent to a second end of the medical packaging, the slip extending from the connection point at the intermediate part towards the second end of the medical packaging and bending and extending towards the openable end of the medical packaging.

By having the first part of the second end of the slip connected with the intermediate part of the medical packaging adjacent to the second end of the medical packaging, the slip may act as a leverage to the medical article. When the user grabs and pulls the slip, the medical article is moved to an approximately maximum distance from the openable end of the medical packaging, corresponding to the distance from the connection point of the slip to the second end of the medical packaging.

The first end of the slip may be connected with the medical packaging at the openable end of the medical packaging.

Having the slip connected with the medical packaging further enhances the user's ability to get hold of the slip before pulling the slip to extract the medical article from the medical packaging.

Further, a first part of the first end of the slip may be connected with the medical packaging and a tab part of the first end of the slip may project further outwards from the medical packaging.

By connecting the slip with the medical packaging and having the slip formed as a tab projecting from the medical packaging, the slip allows for easy opening of the medical packaging by pulling the tab, i.e. the tab part of the first end of the slip, and also provides a grip on the slip to the user for extracting the medical article.

Moreover, the first part of the first end of the slip may be connected with the medical packaging by an adhesive or by means of welding.

By using the same connection methods as when hermetically closing the medical packaging, the connection between the slip and the medical packaging can be performed simultaneous to the closing of the medical packaging.

The slip described above may be made mainly of paper.

Said slip may have been at least partly subjected to a subsequent treatment, such as coating, lamination, printing, dyeing, or any combination thereof.

By using a subsequent treatment of the slip, the slip may be provided with different desired properties, such as protection against liquids, or text can be printed on the slip.

In one embodiment, an additional material may be added to the slip.

By adding an additional material to the slip, the slip may have different desired material properties along its length. Desired effects could be to increase the friction locally between the slip and the medical article by adding droplets made of plastic to the slip, which increases the friction between the medical article and the slip. Another desired effect could be to make the slip part of the same material as that which the medical packaging is made of, providing the tab part of the slip and the medical packaging with the same properties, e.g. welding properties, allowing the tab part of the slip and the medical packaging to be integrated with each other to form the medical packaging when the medical packaging is closed.

Also, the medical article may be a collection device, a urine collection device, a bag, a urology bag, a urine bag, a bladder bag, a leg bag, an infant bag, an ostomy bag, a colostomy bag, an ileostomy bag, a urostomy bag, a catheter, a urology catheter or a tube.

Furthermore, the medical article may be partly made of rubber, latex, silicone, polyethylene, polypropylene, polyurethane, polyvinylchloride, polyvinylidene and methyl acrylate copolymer, ethylene-vinyl acetate, chlorinated polyethylene, polyolefin, styrene-butadiene copolymer, styrene-ethylene-butylene-styrene copolymer and thermoplastic elastomers.

Additionally, the medical packaging may be partly made of rubber, latex, paper, cardboard, polyethylene, polypropylene, polyurethane, polyvinylchloride.

In addition, a guideline or a manual for using the medical article may be printed on the slip, e.g. the leaflet.

The present invention further relates to a method of producing a medical packaging system described above, comprising the steps of:
- providing a medical packaging at least partly made of plastic,
- providing a medical article at least partly made of plastic,
- providing a slip substantially made of a non-plastic material,
- inserting the slip and the medical article into the medical packaging, and
- sealing the medical packaging.

Furthermore, the present invention relates to a method of producing a medical packaging system described above, comprising the steps of:
- providing a medical packaging at least partly made of plastic,
- providing a medical article at least partly made of plastic,
- providing a slip substantially made of a non-plastic material,
- inserting the slip into the medical packaging,
- inserting the medical article into the medical packaging, and
- sealing the medical packaging.

By having a method of producing the medical packaging system according to which the slip, the medical article and the medical packaging may be produced separately and the slip and the medical article may be inserted into the medical packaging one after the other or simultaneously, providing a high degree of flexibility in the production and assembling of the medical packaging system.

Moreover, the present invention relates to a method for extracting the medical article from the medical packaging system described above, comprising the steps of:
- opening the medical packaging at the openable end, and
- extracting the medical article from the medical packaging by grabbing and pulling the slip.

Finally, the present invention relates to a method for extracting the medical article from the medical packaging system described above, comprising the steps of:
- opening the medical packaging at the openable end by grabbing and pulling a tab part of the slip which is connected with and integrally formed with the medical packaging to form a hermetically closed medical packaging,
- breaking the connection between the tab part and the medical packaging, and
- extracting the medical article from the medical packaging by grabbing and pulling the tab part.

### Brief description of the drawings

The invention and its many advantages will be described in more detail below with reference to the accompanying schematic drawings, which for the purpose of illustration show some non-limiting embodiments and in which
Figs. 1a and 1b show cross-sectional views of a medical packaging system in which a slip is arranged underneath or on top of a medical article, respectively,
Figs. 2a and 2b show cross-sectional views of another medical packaging system in which a slip is connected to a medical article by different mechanical attachments,
Fig. 3 shows a cross-sectional view of another medical packaging system in which a slip is connected to a medical article by another mechanical attachment,
Fig. 4 shows a cross-sectional view of yet another medical packaging system in which a slip is connected to a medical article by a mechanical connection,
Fig. 5 shows a cross-sectional view of a medical packaging system in which a slip is partly arranged underneath and on top of a medical article,
Fig. 6 shows a top view of a medical packaging system in which a slip is partly arranged over a second end of a medical article,
Fig. 7 shows a cross-sectional view of a medical packaging system in which a slip is partly arranged over a second end of a medical article and two sides of the medical article,
Fig. 8a shows a cross-sectional view of a medical packaging system in which a slip is also a tab of a medical packaging,
Fig. 8b shows a top view of the medical packaging system of Fig. 8A,
Fig. 9a shows a cross-sectional view of yet another embodiment of the medical packaging system in which a slip is connected to an intermediate part of the medical packaging,
Fig. 9b shows a cross-sectional view of the medical packaging system of Fig. 9A in a second position in which the medical article is partly extracted from the medical packaging, and
Figs. 10 A-C show different medical articles.

All the figures are highly schematic and not necessarily to scale, and they show only those parts which are necessary in order to elucidate the invention, other parts being omitted or merely suggested.

### Detailed description of the invention

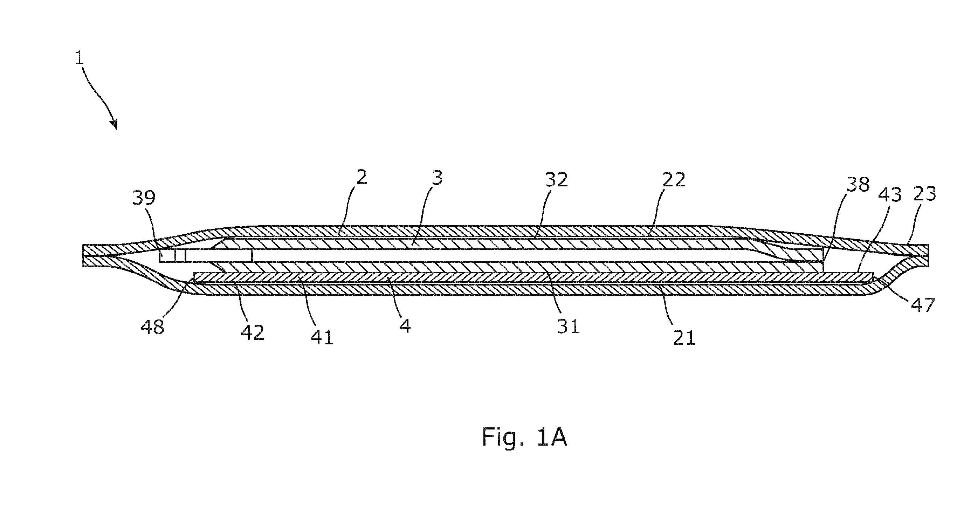

Fig. 1A shows a medical packaging system 1 comprising a medical packaging 2 which is at least partly made of plastic and encloses a medical article 3 which is also at least partly made of plastic, resulting in the potential risk that the article may get stuck within the medical packaging when a user of the article tries to remove the article from the packaging. The medical packaging system 1 further comprises a slip 4, such as a leaflet, substantially made of a non-plastic material, arranged at least between a first surface 31 of the medical article 3 and a first inner face 21 of the medical packaging 2.

By arranging the slip 4 substantially made of a non-plastic material between the medical article 3 and the medical packaging 2, the friction between the plastic materials of the medical article 3 and the medical packaging 2 is substantially reduced, making extraction of the medical article 3 from the medical packaging 2 much easier for the user.

Further, by having the slip 4 arranged at least between the first surface 31 of the medical article 3 and the first inner face 21 of the medical packaging 2, the user can choose to grab and pull the medical article 3 and/or grab and pull the slip 4 to extract the medical article 3. When grabbing and pulling the slip 4, the slip 4 then acts as a carrier to the medical article 3 and extracts the medical article 3, together with the slip 4, out of the medical packaging 2, hence reducing the friction between the first inner face 21 of the medical packaging 2 and the first surface 31 of the medical article 3. Consequently, the amount of friction to be overcome by the user when extracting the medical article 3 from the medical packaging 2 is substantially reduced. Alternatively, the user can grab and pull the medical article 3, so that the medical article 3 slides on the slip 4 when removed from the medical packaging 2.

The slip 4 is hereby used for reducing the friction between the medical packaging 2 and the medical article 3. Thus, the slip 4 is preferably made of a material, such as primarily paper, reducing the friction between the medical packaging 2 made of plastic and the medical article 3 made of plastic.

Usually, a leaflet is accompanying the medical article 3 in the medical packaging 2, instructing the user how to use the medical article 3. According to this invention, the slip 4 is preferably the leaflet accompanying the medical article 3 in the medical packaging 2. The accompanying leaflet serving as the slip 4 makes it possible for example to reduce the extra costs of producing the slip 4 as an additional component besides the medical article 3 and the leaflet accompanying the medical article 3. By using the accompanying leaflet as the slip 4 further reduces the volume of the medical packaging 2 and saves production costs, since the medical packaging 2 will comprise fewer components, i.e. the medical article 3 and the leaflet, and the medical packaging 2 can thus be produced from less material covering a smaller volume than if there were to be more components in the medical packaging 2.

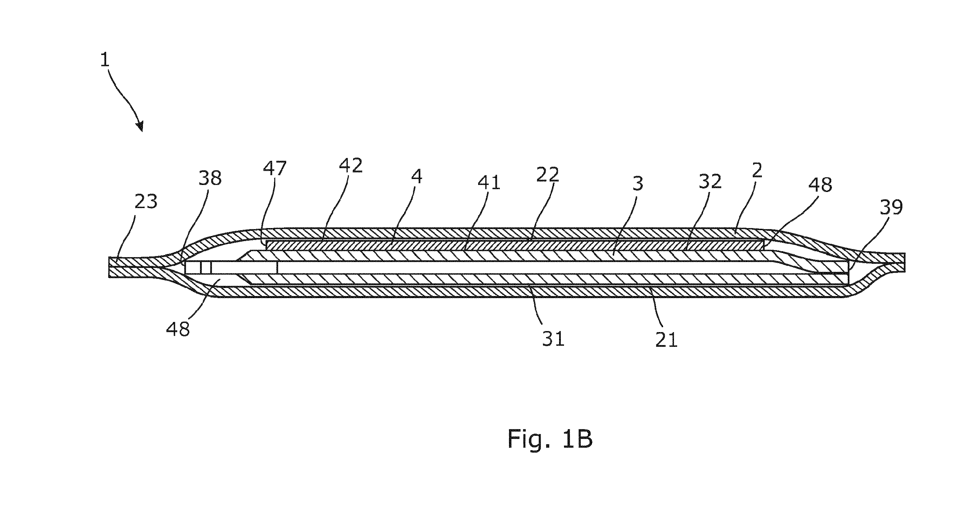

As shown in Fig. 1A, the slip 4 can be arranged underneath the medical article 3, between the first surface 31 of the medical article 3 and the first inner face 21 of the medical packaging 2. Alternatively, the slip 4 can be arranged on top of the medical article 3, between a second surface 32 of the medical article 3 and a second inner face 22 of the medical packaging 2, as shown in Fig. 1B.

The medical packaging 2 is preferably hermetically closed by welding or adhering, such as glueing, hence securing an environment in the medical packaging 2. The environment is preferably substantially sterile or more preferably sterile. However, at least one end is made as an openable end 23 of the medical packaging 2 for the user to break the seal and remove the medical article 3 from the medical packaging 2 through the openable end 23.

As seen in Figs. 1A and 2A-9B, the slip 4 projects from a first end 38 of the medical article 3 towards the openable end 23 of the medical packaging 2. By having the slip 4 projecting from the first end 38 of the medical article 3 in a direction of the openable end 23, a projected area 43 of the slip 4 is created which the user can grab and pull. The created projected area 43 aids the user in getting a hold of the slip 4 before extracting the slip 4 and the medical article 3 from the medical packaging 2.

With respect to the slip 4 acting as the carrier for the medical article 3 when extracting the medical article 3 by grabbing and pulling the slip 4, the slip 4 is connected with the medical article 3 by friction. Furthermore, when using the slip 4 as the carrier for the medical article 3, the friction between the slip 4 and the medical article 3 is higher than the friction between the medical article 3 and the medical packaging 2. By connecting the medical article 3 and the slip 4 by means of friction, the slip 4 and the medical article 3 interacts and creates a simple connection between them. The simple connection allows, in an easy way, the medical article 3 to follow the slip 4 out of the medical packaging 2 when the user grabs and pulls the slip 4 to extract the medical article 3 from the medical packaging 2. The slip 4 is thus made of a first material on a first surface 41 facing the medical article 3, which together with the medical article 3 has higher friction than a second material of the slip 4 on a second surface 42 of the slip 4 which is facing the medical packaging 2.

Instead of having high friction between the slip 4 and the medical article 3, the friction between the slip 4 and the medical article 3 is lower than the friction between the medical article 3 and the medical packaging 2. By having lower friction between the slip 4 and the medical article 3 than between the medical article 3 and the medical packaging 2, the medical article 3 is extracted by grabbing and pulling the medical article 3 itself. This results in the user having to use less force when extracting the medical article 3 than if he/she has to overcome the friction between the medical article 3 and the medical packaging 2 without the slip 4 arranged between the medical article 3 and the medical packaging 2.

To further strengthen the connection between the slip 4 and the medical article 3, the slip 4 may be connected with the medical article 3 by an adhesive or by means of welding. By using such a connection between the medical article 3 and the slip 4, a greater force can be used when pulling the slip 4 to extract the medical article 3 from the medical packaging 2. The slip 4 can also be pulled in more arbitrary directions compared to the simple connection by means of friction, since the connection is not dependent on the interaction between the surfaces of the medical article 3 and the slip 4 creating the simple friction connection. Thus, the requirement of the user and the user's ability to grab and pull the slip 4 is reduced. Additionally, the slip 4 can be made from a single material resulting in low friction between the slip 4 and the medical packaging 2 compared to the slip 4 and the medical article 3 being connected by friction and two materials being used to make the slip 4.

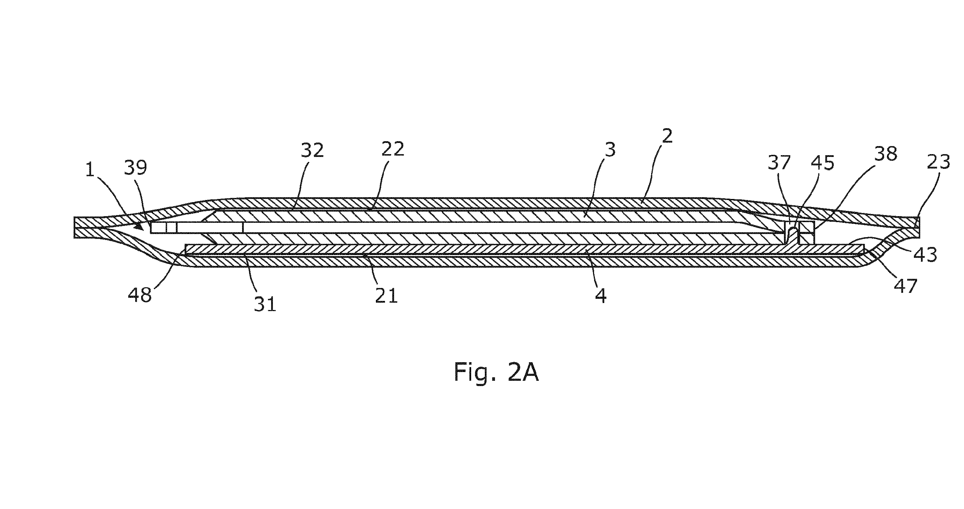

In Fig. 2A, an alternative connection between the slip 4 and the medical article 3 is shown as a connection where a mechanical attachment connects the slip 4 with the medical article 3. By using the mechanical attachment between the slip 4 and the medical article 3, several types of mechanical connections can be incorporated with the slip 4 and/or the medical article 3 to make a secure connection between the slip 4 and the medical article 3 when the user grabs and pulls the slip 4 to extract the medical article 3 from the medical packaging 2.

As shown in Fig. 2A, the mechanical attachment is a projection 45 on the first surface 41 of the slip 4 facing the medical article 3 and being adapted to engage an opening 37 in the medical article 3. By using the projection 45 on the slip 4 projecting into and engaging the opening 37 in the medical article 3, a secure attachment between the slip 4 and the medical article 3 is achieved. The opening 37 in the medical article 3 is for example an already existing opening in the medical article 3 used for hanging the medical article on a stand, hence eliminating the need for creating an opening in the medical article 3. The opening 37 in the medical article 3 can also be a depression or a slit in the medical article 3.

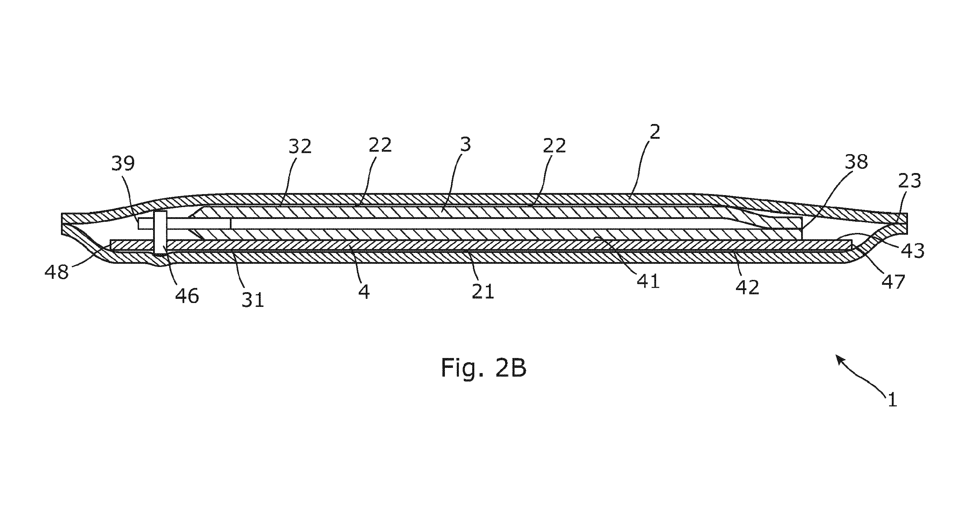

As seen in Fig. 2B, the projection is a projecting part 36 of the medical article 3 and consequently, the opening 46, the depression or the slit is arranged in the slip 4. Hence, the mechanical attachment is made of an already existing projection 36 in the medical article 3.

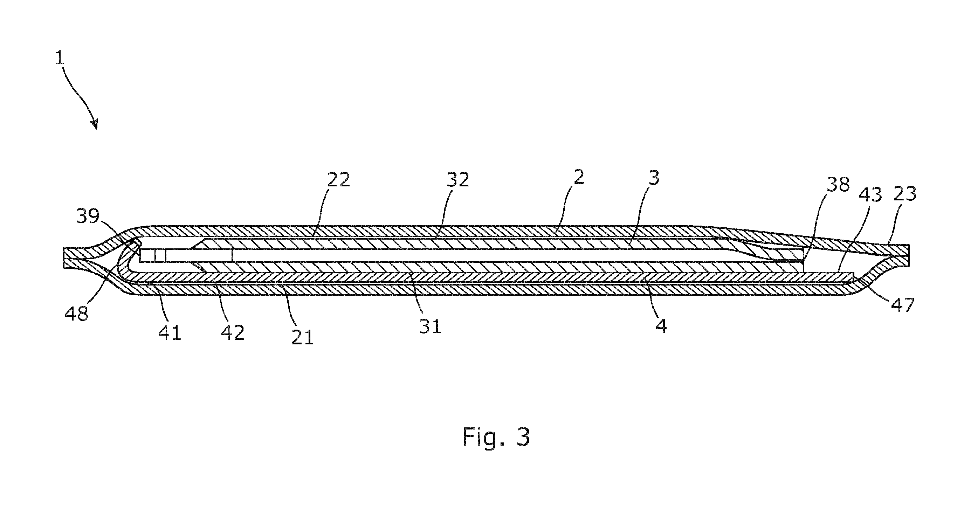

In Fig. 3, the mechanical attachment is formed as a part of a second end 48 of the slip 4 and angled in relation to the first surface 41 of the slip 4 facing the medical article 3, with an angle of 0-90 degrees. The slip 4 has a first end 47 and the first end 47 is closer to the openable end 23 than the second end 48 of the slip 4. The mechanical attachment forming part of the second end 48 of the slip 4, said mechanical attachment being angled in relation to the first surface 41 of the slip 4, results in the slip 4 having an elevated edge abutting the medical article 3 when the user grabs and pulls the slip 4 to extract the medical article 3 from the medical packaging 2. The mechanical attachment in the form of an elevated edge acts as a stop which prevents the medical article 3 from remaining inside the medical packaging 2 when the user grabs and pulls the slip 4 to extract the medical article 3 from the medical packaging 2.

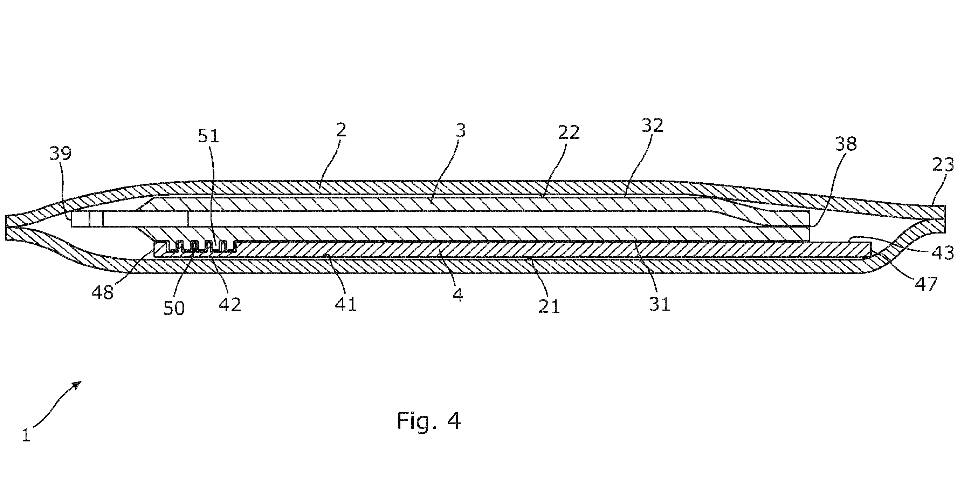

As shown in Fig. 4, the mechanical attachment comprises a first connection part 50 and a second connection part 51, the first connection part 50 having a shape corresponding to a shape of the second connection part 51. By using two connection parts 50, 51 having corresponding shapes, a wide range of mechanical attachments with different shapes can be used. The first connection part 50 is arranged on the first surface 41 of the slip 4 facing the medical article 3 and the second connection part 51 is arranged on the first 31 surface of the medical article 3, or vice versa. For example, the shape of the second connection part 51 is a design feature, such as corrugations, a tap or lid and is arranged on the first surface 31 of the medical article 3. The first connection part 50 of the slip 4 is then made to correspond to the second connection part 51 of the medical article 3 in order to create the mechanical attachment.

The first connection part 50 of the slip 4 and the second connection part 51 of the medical article 3 are alternatively arranged so that when the user grabs and pulls the slip 4 to extract the medical article 3 from the medical packaging 2, the first connection part 50 and the second connection part 51 interconnects and forms the mechanical attachment. This may be achieved by the first connection part 50 being formed at the second end 48 of the slip 4 so that when the user grabs and pulls the slip 4, the first connection part 50 of the slip 4 interconnects with or "catches" a projection or opening on the first surface 31 of the medical article 3.

Regarding the different connection means, the slip 4 and the medical article 3 are releasably connected with each other or alternatively the slip 4 and the medical article 3 are breakably connected. By having the releasable or breakable connection between the slip 4 and the medical article 3, the slip 4 is separated from the medical article 3 or upon extraction from the medical packaging 2, allowing the user to easily read the slip 4. Alternatively, the releasable or breakable connection allows the user to remove the slip 4 from the medical article 3 so the slip 4 is not disturbing the use of the medical article 3. The disengagement of the slip 4 and the medical article 3 also allows the slip 4 to be reused or saved.

With regard to the arrangement of the slip 4 between the first surface 31 of the medical article 3 and the first inner face 21 of the medical packaging 2, the first surface 31 of the medical article 3 has an area, and the first surface 41 of the slip 4 facing the medical article 3 has an area. The area of the slip 4 overlaps at least 10% of the area of the first surface 31 of the medical article 3, preferably at least 30%, more preferably at least 50%, and most preferably between 80% and 100% of the area of the first surface 31 of the medical article 3. The overlap between the slip 4 and the medical article 3 depends on for example the slip 4 being folded before being arranged between the medical article 3 and the medical packaging 2, allowing the medical article 3 to still be visible through the medical packaging 2. The overlap may also depend on the production costs of the slip 4, i.e. a slip 4 with a small first surface 41 facing the medical article 3 is cheaper to produce than a slip 4 with a large first surface 41 facing the medical article 3.

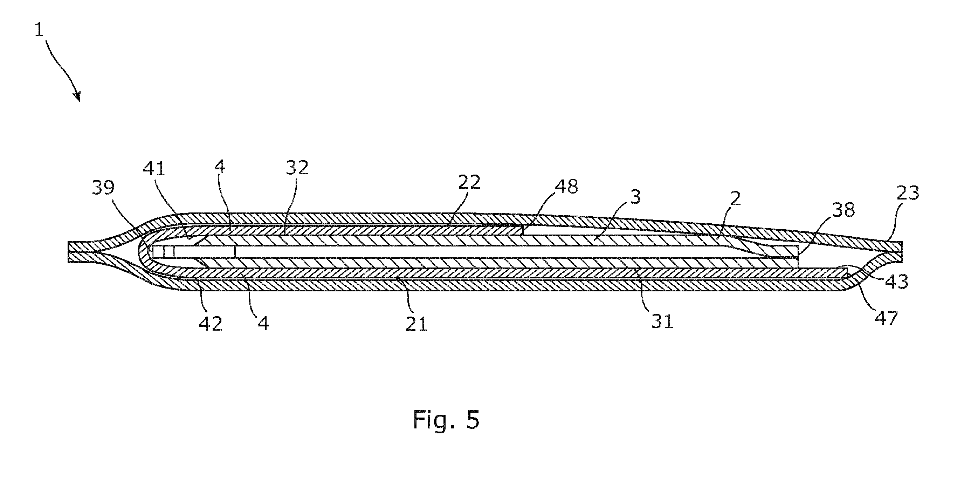

As seen in Fig. 5, the slip 4 is partly arranged between the first inner face 21 of the medical packaging 2 and the first surface 31 of the medical article 3 and partly arranged between the second inner face 22 of the medical packaging 2 and the second surface 32 of the medical article 3. By partly arranging the slip 4 both between the medical packaging 2 and the first 31 and lower surface of the medical article 3 and partly between the medical packaging 2 and the second 32 and upper surface of the medical article 3, the friction between the medical article 3 and the medical packaging 2 is further reduced compared to the slip 4 being arranged on one side only of the medical article 3. Thus, the slip 4 partly envelops the medical article 3, allowing the slip 4 to act as an extraction device when the user pulls the slip 4 to extract the medical article 3 from the medical packaging 2. By having the slip 4 partly arranged underneath and on top of the medical article 3, the slip 4 guides the medical article 3 out of the medical packaging 2 without using one of the previously described connection means such as friction, an adhesive agent, welding or similar mechanical attachment between the slip 4 and the medical article 3.

In another embodiment, the area of the first surface 41 of the slip 4 facing the medical article 3 covers at least 10% of the area of the second surface 32 of the medical article 3, preferably at least 30%, more preferably at least 50%, most preferably between 80% and 100% of the area of the second surface 32 of the medical article 3. By allowing the slip 4 to overlap the medical article 3 in an arbitrary way and to be arranged between the second inner face 22 of the medical packaging 2 and the second surface 32 of the medical article 3, the slip 4 can have different shapes underneath and on top of the medical article 3. Furthermore, when packing the medical article 3 in the medical packaging 2, the slip does not have to be arranged in a very precise manner in order to obtain the desired effect. In addition, the shape of the slip 4 can for example be chosen to match a contour of the medical article 3, providing maximum grip and guidance of the medical article 3 when the user extracts the medical article 3 from the medical packaging 2.

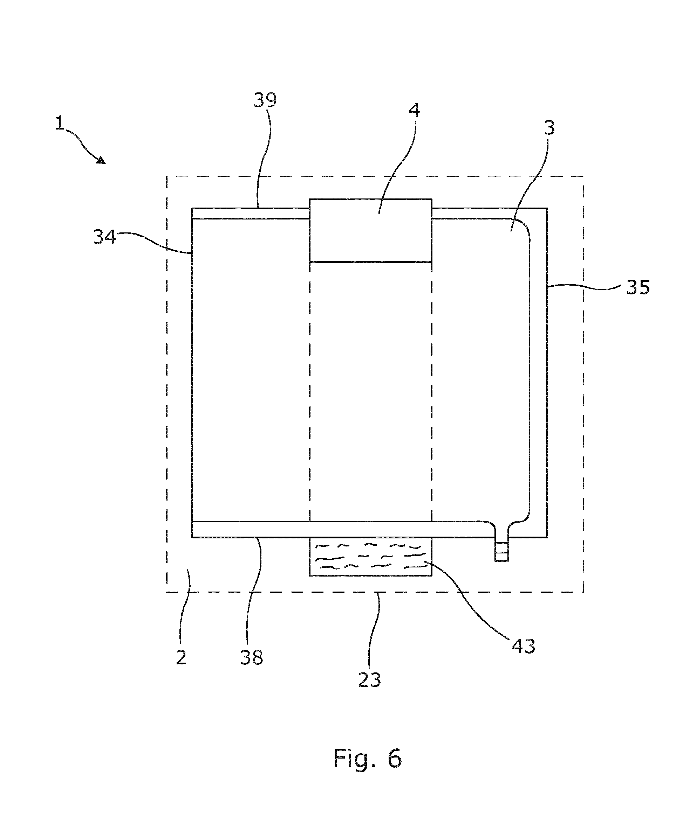

Another shape of the slip 4 that partly envelops the medical article 3 is seen in Fig. 6, in which the second end 39 is arranged further away from the openable end 23 than the first end 38 of the medical article 3 and the slip 4 is arranged partly covering the second end 39 of the medical article 3. Fig. 6 shows the projected area 43 of the slip 4 allowing the user to easily grab and get hold of the slip 4. Further, Fig. 6 shows the slip 4 being partly arranged underneath the medical article 3 and partly covering the second end 39 of the medical article 3. The slip 4 is thus arranged underneath, over the second end 39 and on top of the medical article 3. By having the slip 4 partly covering the second end 39 of the medical article 3, the slip 4 forms a "hook" and the slip 4 thereby acts an extraction device capable of securing the slip 4 to the medical article 3 and capable of guiding the medical article 3 out of the medical packaging 2 when the user grabs and pulls the slip 4.

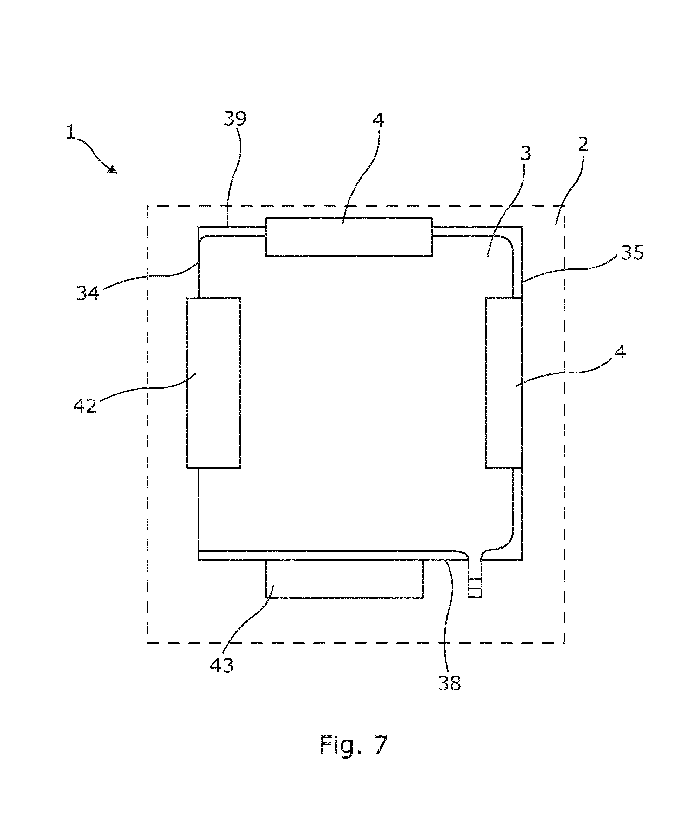

Another shape of the slip 4 arranged underneath, over the second end 39 and on top of the medical article 3 is seen in Fig. 7. The medical article 3 has a first side 34 and a second side 35 and the slip 4 is partly covering the first side 34 and the second side 35 of the medical article 3 and is partly covering the second end 39 of the medical article 3. By having the slip 4 partly covering both sides 34, 35 and partly covering the second end 39 of the medical article 3, the slip 4 is formed as a "pouch". The medical article 3 is thereby retained further, securing and guiding the medical article 3 when the user grabs and pulls the slip 4 to extract the medical article 3 from the medical packaging 2, than if the slip 4 only partly covers the second end 39 of the medical article 3.

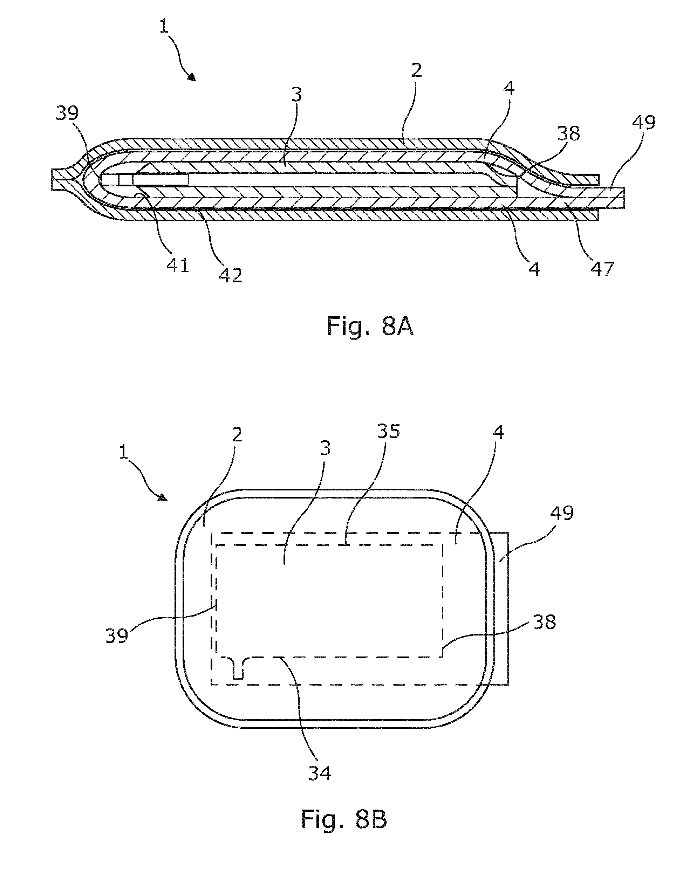

Furthermore, another shape of the slip 4 is shown in Figs. 8A -8B in which the slip 4 envelops the medical article 3. Fig. 8A shows a cross-sectional view of the medical packaging system 1, and Fig. 8B shows a top view of the medical packaging system 1. By having the slip 4 enveloping the medical article 3, the friction between the medical article 3 and the medical packaging 2 is completely eliminated since the medical article 3 and the medical packaging 2 no longer have any contacting surfaces. The friction thus depends on the interaction between the material of the medical packaging 2 and the material of the slip 4. Thus, the user will - with even less force than previously described - be able to extract the medical article 3 from the medical packaging 2.

The medical article 3 is most often used by elderly people or disabled people who most often are in a situation where they have to perform replacement of the medical article 3 quickly. To further aid such users in extracting the medical article 3, the slip 4 may be connected with the medical packaging 2 at the openable end 23 of the medical packaging 2. Hereby, the user's ability to grab the slip 4 before pulling the slip 4 to extract the medical article 3 from the medical packaging 2 is greatly improved.

To even further help the user extract the medical article 3 from the packaging 2, a first part of the first end 47 of the slip 4 is connected with the medical packaging 2 and a tab part 49 of the first end 47 of the slip 4 projects further outwards from the medical packaging 2 as seen in Figs. 8A and 8B. By connecting the slip 4 with the medical packaging 2 and having the slip 4 formed as a tab of the medical packaging 2, the slip 4 allows for the user to both open the medical packaging 2 by pulling the tab, i.e. the tab part 49 of the first end of the slip 4, and at the same time provides the user with a grip on the slip 4 for a subsequent extraction of the medical article 3 from the medical packaging 2.

The connection of the first part of the first end 47 of the slip 4 with the medical packaging 2 is preferably achieved by using an adhesive or by welding the first part of the first end 47 of the slip 4 with the medical packaging 2. By using the same connection means as when hermetically closing the medical packaging 2, the connection between the slip 4 and the medical packaging 2 can be performed simultaneous to hermetically closing the medical packaging 2.

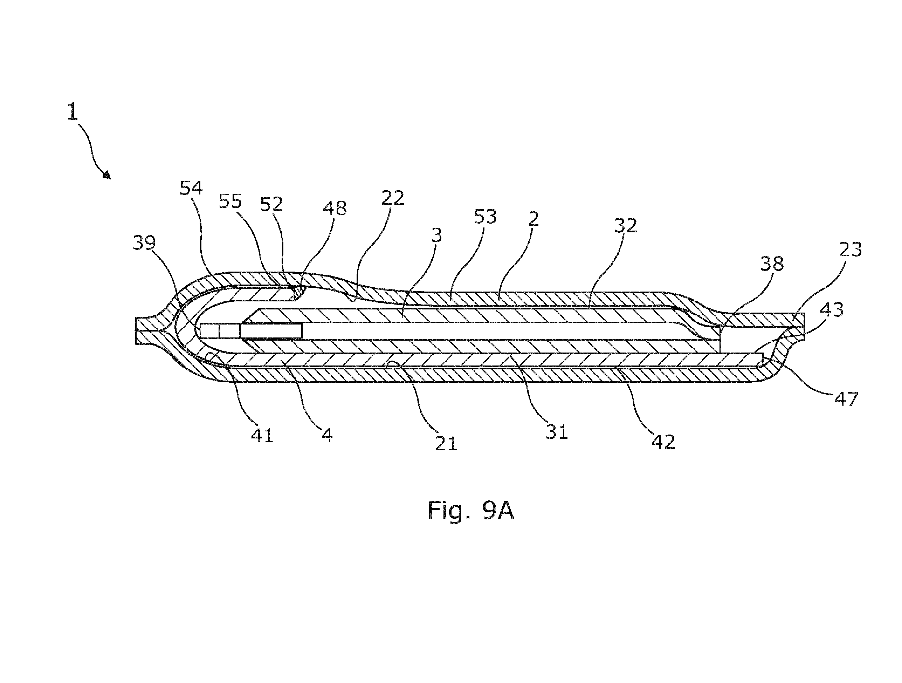

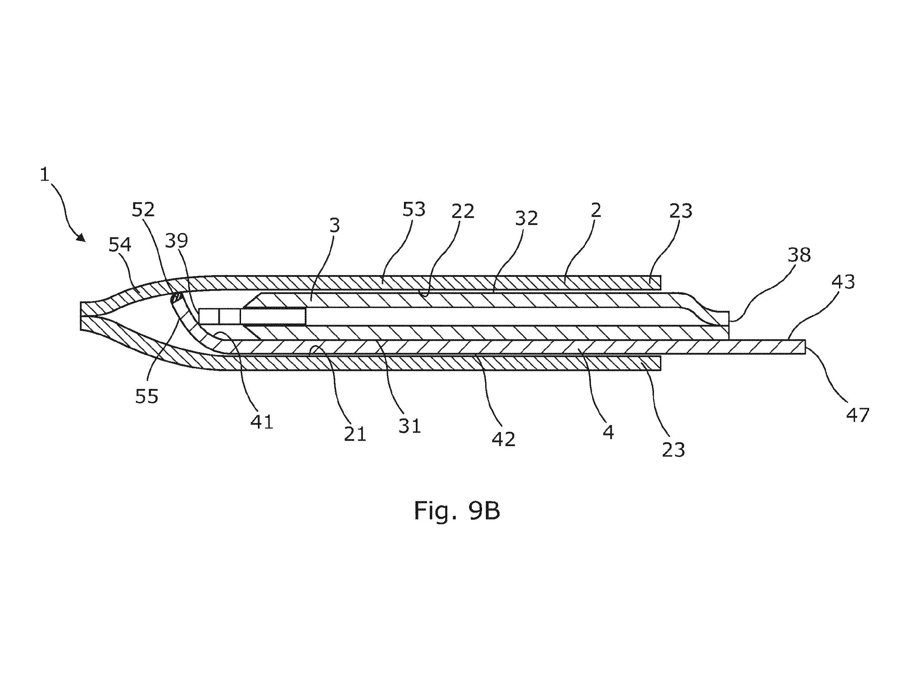

An alternative way of aiding in the extraction of the medical article 3 is shown in Fig. 9A, in which a first part 55 of the second end 48 of the slip 4 is connected in a connection point 52 with an intermediate part 53 of the medical packaging 2, adjacent to a second end 54 of the medical packaging 2. The slip 4 extends from the connection point 52 on the intermediate part 53 towards the second end 54 of the medical packaging 2 and bends and extends towards the openable end 23 of the medical packaging 2. By having the first part 55 of the second end 48 of the slip 4 connected with the intermediate part 53 of the medical packaging 2 adjacent to the second end 54 of the medical packaging 2, the slip 4 acts as a leverage to the medical article 3. When the user grabs and pulls the slip 4, the medical article 3 is moved to an approximately maximum distance from the openable end 23 of the medical packaging 2, corresponding to the distance from the connection point 52 of the slip 4 to the second end 54 of the medical packaging 2 as shown in Fig. 9B.

With respect to the slip 4 and the material of the slip 4, the slip 4 is partly or entirely made of paper or similar material and may be at least partly subjected to a subsequent treatment, such as coating, lamination, printing, dyeing, or any combination thereof. By subjecting the slip 4 to a subsequent treatment, the slip 4 can be provided with different desired properties, such as protection against liquids, or text can be printed on the slip 4, resulting in the slip serving as the leaflet. The printed text is preferably a guideline or a manual for using the medical article, as can be seen in Fig. 6.

Additionally, the slip 4 may have an additional material added or coated onto the slip 4. By adding an additional material to the slip 4, the slip 4 is given different desired material properties along its length. Desired effects could be to increase the friction locally between the slip 4 and the medical article 3 by adding droplets made of plastic to the slip 4, which increases the friction between the medical article 3 and the slip 4, allowing the slip 4 and the medical article 3 to be connected by friction. Another desired effect is to form the slip 4 as an integral part of the medical packaging 2 when the medical packaging 2 is closed, which preferably requires the slip 4 and the medical packaging 2 to be made of the same material.

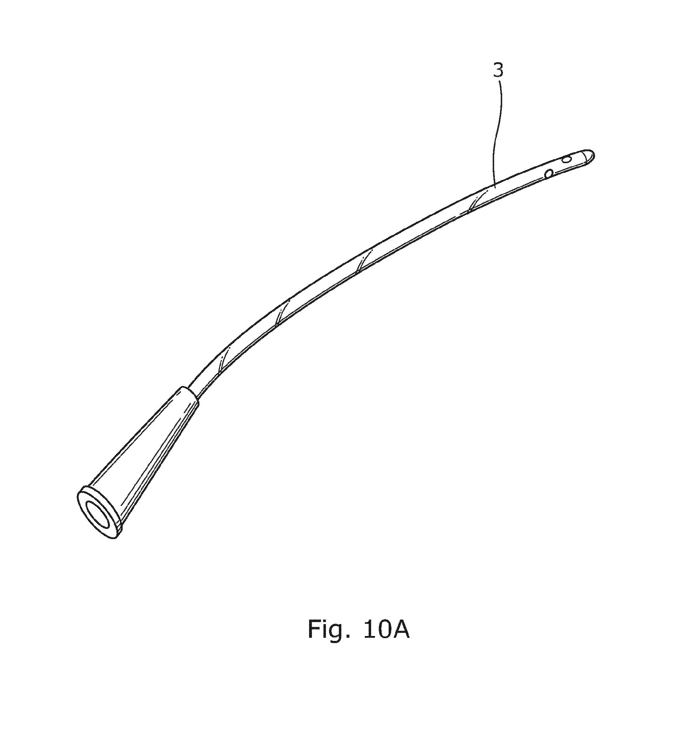

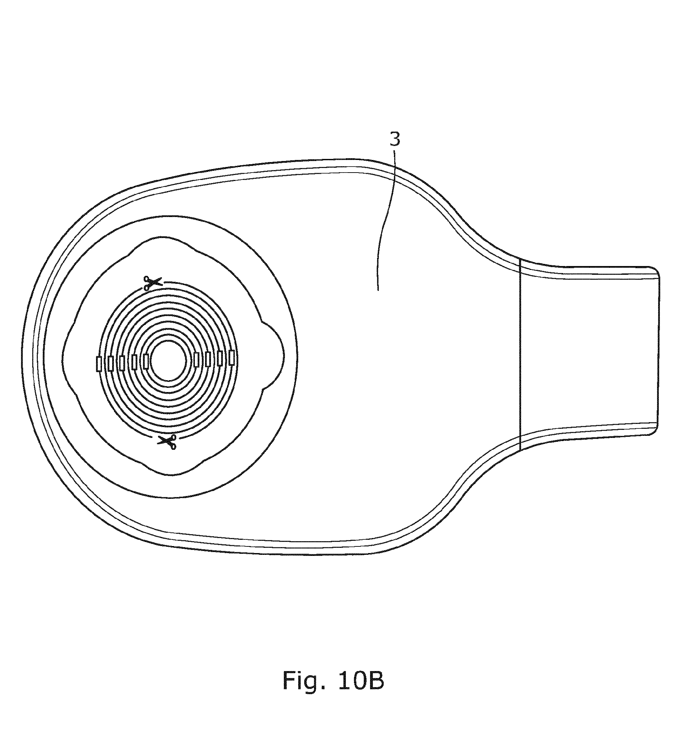

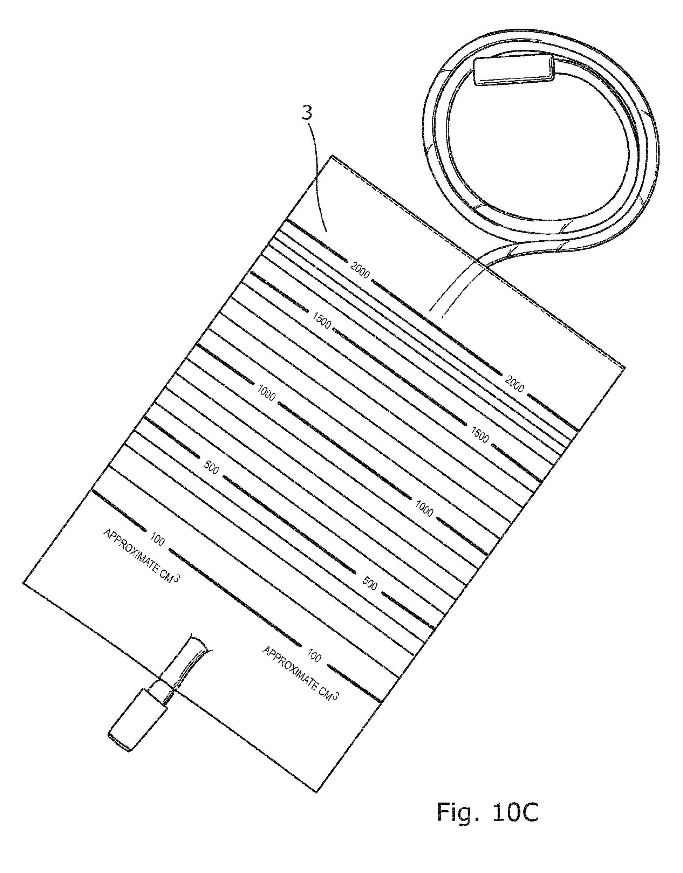

The medical article 3 is preferably a collection device, a urine collection device, a bag, a urology bag, a urine bag (shown in Fig. 10A), a bladder bag, a leg bag, an infant bag, an ostomy bag (shown in Fig. 10B), a colostomy bag, an ileostomy bag, a urostomy bag, a catheter (shown in Fig. 10C), a urology catheter or a tube. The medical article 3 is preferably partly made of rubber, latex, silicone, polyethylene, polypropylene, polyurethane, polyvinylchloride, polyvinylidene and methyl acrylate copolymer, ethylene-vinyl acetate, chlorinated polyethylene, polyolefin, styrene-butadiene copolymer, styrene-ethylene-butylene-styrene copolymer, thermoplastic elastomers or any combination thereof.

The medical packaging 2 is preferably partly made of rubber, latex, paper, cardboard, polyethylene, polypropylene, polyurethane, polyvinylchloride or any combination thereof.

When producing the medical packaging system 1, the medical packaging 2, the medical article 3 and the slip 4 is most often produced by different machines in a production facility. This is due to a difference in construction of the medical packaging 2, the medical article 3 and the slip 4 and the materials used. Following the production of the three individual components, the medical packaging 2, the medical article 3 and the slip 4, the three individual components are assembled. The assembling is performed by collecting the medical article 3 and the slip 4 and inserting them into the medical packaging 2. When the medical article 3 and the slip 4 have been inserted into the medical packaging 2, the medical packaging 2 is sealed to protect the medical article 3 from the surrounding environment. Instead of collecting the medical article 3 and the slip 4 and inserting them into the medical packaging 2, the medical article 3 and the slip 4 may be inserted into the medical packaging one after the other. In another embodiment, the medical packaging is a two-part packaging comprising a base part and a lid part or top film and a bottom film. The slip 4 is then arranged on the base part or bottom film and subsequently or simultaneously the medical article 3 is arranged on the base part or bottom film. Subsequently, the lid or top film is arranged on top of the base part or bottom film and a hermetical sealing process takes place.

When extracting the medical article 3 from the medical packaging of Figs. 1A-7 and 9A-9B, the user will preferably grab the medical packaging 2 at the openable end 23 and open the medical packaging 2 by breaking the hermetical seal. In Figs. 1A, 2A-7 and 9A-9B, the user will then grab and pull the slip 4 to extract the medical article 3. In Fig. 1B, the user will pull the medical article 3 sliding on the slip 4. Alternatively, as shown in Figs. 8A and 8B, the user will open the medical packaging 2 by pulling the tab part 49 of the slip 4 forming part of the the hermetical closing of the medical packaging 2.

When the user has opened the medical packaging 2 of Figs. 2A, 2B and 4 and grabbed and pulled the slip, the user releases the connection between the slip 4 and the medical packaging 2.

A slip is to be understood as a slipcover, a sheet, a strip or a paper-thin component with similar dimensions as paper normally accompanying a medical article in a medical packaging.

Although the invention has been described in the above in connection with preferred embodiments of the invention, it will be evident for a person skilled in the art that several modifications are conceivable without departing from the invention as defined by the following claims.

## Claims

1. A medical packaging system (1), comprising:
- a medical packaging (2) at least partly made of plastic and having a first inner face (21) and a second inner face (22) and an openable end (23), enclosing
- a medical article (3) at least partly made of plastic and having a first surface (31) and a second surface (32),
further comprising a slip (4) substantially made of a non-plastic material, arranged at least between the first surface of the medical article and the first inner face of the medical packaging.

2. A medical packaging system according to claim 1, wherein the slip projects from a first end (38) of the medical article towards the openable end of the medical packaging.

3. A medical packaging system according to claim 1 or 2, wherein the slip is connected with the medical article by friction and the friction between the slip and the medical article is higher than the friction between the first surface and/or the second surface of the medical article and the first inner face and/or the second inner face of the medical packaging.

4. A medical packaging system according to claim 1 or 2, wherein a friction between the slip and the medical article is lower than a friction between the first surface and/or second surface of the medical article and the first inner face and/or second inner face of the medical packaging.

5. A medical packaging system according to any of the preceding claims, wherein the slip is connected with the medical article by a mechanical attachment.

6. A medical packaging system according to claim 5, wherein the mechanical attachment is a projection (45) on a first surface (41) of the slip facing the medical article, which projection is adapted to engage with a depression, a slit, or an opening (37) on the medical article, or vice versa.

7. A medical packaging system according to any of the preceding claims, wherein the slip is releasably connected with the medical article.

8. A medical packaging system according to any of the preceding claims, wherein the first surface of the slip facing the medical article has an area, and the first surface of the medical article has an area, the area of the slip covering at least 10% of the area of the first surface of the medical article, preferably at least 30%, more preferably at least 50%, most preferably between 80% and 100%.

9. A medical packaging system according to any of the preceding claims, wherein the slip is partly arranged between the first inner face of the medical packaging and the first surface of the medical article and partly arranged between the second inner face of the medical packaging and the second surface of the medical article.

10. A medical packaging system according to claim 9, wherein the medical article has a first end (38) and a second end (39), the first end of the medical article being arranged closer to the openable end of the medical packaging than the second end of the medical article and the slip being arranged partly covering the second end of the medical article.

11. A medical packaging system according to claim 9 or 10, wherein the medical article has a first side (34) and a second side (35) and the slip is partly covering the first and the second side of the medical article and partly covering the second end of the medical article.

12. A medical packaging system according to claims 9, 10 or 11, wherein a first part (55) of the second end of the slip is connected in a connection point (52) with an intermediate part (53) of the medical packaging, adjacent to a second end (54) of the medical packaging, the slip extending from the connection point at the intermediate part towards the second end of the medical packaging and bending and extending towards the openable end of the medical packaging.

13. A medical packaging system according to any of the preceding claims, wherein the medical article is a collection device, a urine collection device, a bag, a urology bag, a urine bag, a bladder bag, a leg bag, an infant bag, an ostomy bag, a colostomy bag, an ileostomy bag, a urostomy bag, a catheter, a urology catheter or a tube.

14. A method of producing a medical packaging system according to any of the preceding claims, comprising the steps of:
- providing a medical packaging at least partly made of plastic,
- providing a medical article at least partly made of plastic,
- providing a slip substantially made of a non-plastic material,
- inserting the slip and the medical article into the medical packaging, and
- sealing the medical packaging.

15. A method for extracting the medical article from the medical packaging system according to claims 1-13 comprising the steps of:
- opening the medical packaging at the openable end, and
- extracting the medical article from the medical packaging by grabbing and pulling the slip.
